(19) Europäisches Patentamt
European Patent Office
Office européen des brevets

(11) Veröffentlichungsnummer: 0 286 927
A2

(12) # EUROPÄISCHE PATENTANMELDUNG

(21) Anmeldenummer: 88105331.8

(22) Anmeldetag: 01.04.88

(51) Int. Cl.[4]: C07D 207/08 , C07D 401/12 , C07D 405/12 , C07D 405/14 , C07K 5/06 , C07K 5/08 , A61K 31/40 , A61K 31/44 , A61K 31/505 , A61K 37/02

Patentansprüche für folgende Vertragsstaaten: ES + GR.

(30) Priorität: 11.04.87 DE 3712364

(43) Veröffentlichungstag der Anmeldung: 19.10.88 Patentblatt 88/42

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Henning, Rainer, Dr.
Rotenhofstrasse 31
D-6234 Hattersheim am Main(DE)
Erfinder: Hock, Franz, Dr.
Altstadt 19
D-6110 Dieburg(DE)
Erfinder: Urbach, Hansjörg, Dr.
Le Lavandoustrasse 41
D-6242 Kronberg/Taunus(DE)

(54) **Neue Pyrrolidin-2-(1,3-dicarbonyl)-Derivate, Verfahren zu ihrer Herstellung, sie enthaltende Mittel sowie deren Verwendung.**

(57) Die Erfindung betrifft Pyrrolidin-Derivate der allgemeinen Formel

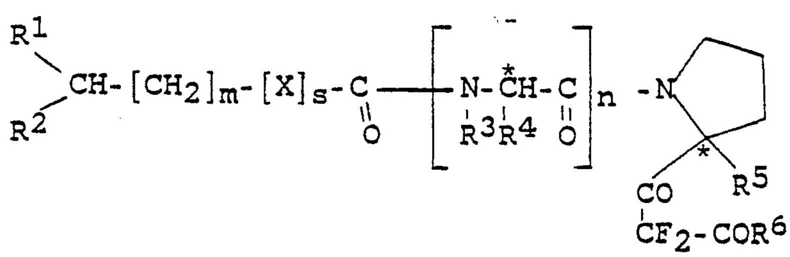

in welcher
$R^1$, $R^2$, $R^3$, $R^4$, $R^5$ und $R^6$ die in der Beschreibung angegebene Bedeutung haben, X Sauerstoff, Imino oder Alkylimino bedeutet, m = 0-5, n = 0-2 und s = 0 oder 1 ist, Verfahren zu ihrer Herstellung, sie enthaltende Mittel sowie deren Verwendung.

EP 0 286 927 A2

# Neue Pyrrolidin-2-(1,3-dicarbonyl)-Derivate, Verfahren zu ihrer Herstellung, sie enthaltende Mittel sowie deren Verwendung

Die Erfindung betrifft Pyrrolidin-Derivate der allgemeinen Formel I

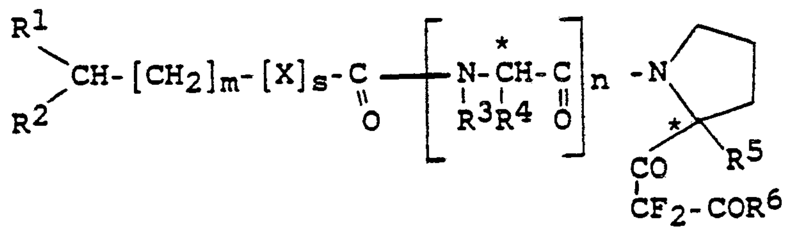

(I)

in welcher

$R^1$ Wasserstoff; $(C_1-C_6)$-Alkyl oder $(C_6-C_{12})$-Aryl, das gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino oder ein ($C_1$ oder $C_2$)-Alkylendioxy substituiert ist, bedeutet;

$R^2$ Wasserstoff; $(C_1-C_6)$-Alkyl; $(C_6-C_{12})$-Aryl; $C_6-C_{12})$-Aryloxy; $(C_7-C_{13})$-Aroyl; Hydroxy oder $(C_1-C_4)$-Alkoxy bedeutet, wobei Aryl, Aryloxy und Aroyl jeweils gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist; oder

$R^1$ und $R^2$ zusammen für Benzyliden stehen, worin der Phenylring gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino, und Di-$(C_1-C_4)$-alkylamino, oder ein ($C_1$ oder $C_2$)-Alkylendioxy substituiert ist;

$R^3$ Wasserstoff; $(C_1-C_6)$-Alkyl; $(C_6-C_{12})$-Aryl-$(C_1-C_6)$-alkyl; $(C_5-C_9)$-Cycloalkyl; Indanyl oder Tetrahydronaphthyl bedeutet;

$R^4$ Wasserstoff; $(C_1-C_6)$-Alkyl, das gegebenenfalls durch Amino, $(C_1-C_6)$-Acylamino, insbesondere $(C_1-C_6)$-Alkanoylamino oder Boc-NH, oder Benzoylamino monosubstituiert sein kann; $(C_2-C_6)$-Alkenyl; $(C_5-C_9)$-Cycloalkyl; $(C_5-C_9)$-Cycloalkenyl; $(C_5-C_7)$-Cycloalkyl$(C_1-C_4)$-alkyl; $(C_6-C_{12})$-Aryl oder teilhydriertes $(C_6-C_{12})$-Aryl, die jeweils durch einen, zwei oder drei gleiche oder verschiedene Reste aus der Reihe $(C_1-C_4)$-Alkyl, $(C_1-C_2)$-Alkoxy und Halogen substituiert sein können; $(C_6-C_{12})$-Aryl-$(C_1-C_4)$-alkyl oder $(C_7-C_{13})$-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1-9 Ringatome Kohlenstoffatome und 1 bis 2 Ringatome Schwefel-oder Sauerstoffatome und/oder wovon 1 bis 4 Ringatome Stickstoffatome darstellen, oder, falls von vorstehenden Definitionen noch nicht umfaßt, die gegebenenfalls geschützte Seitenkette einer natürlich vorkommenden α-Aminosäure bedeutet; oder

$R^3$ und $R^4$ zusammen für $-[CH_2]_p$-stehen, worin p = 3, 4 oder oder 5 ist und worin eine Methylengruppe durch S oder O ersetzt sein kann;

$R^5$ Wasserstoff; $(C_1-C_6)$-Alkyl; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^6$ $(C_1-C_8)$-Alkyl; $(C_6-C_{12})$-Aryl; $(C_6-C_{12})$-Aryl-$(C_1-C_6)$-alkyl; Hydroxy; $(C_1-C_8)$-Alkoxy; $(C_6-C_{12})$-Aryloxy oder $(C_6-C_{12})$-Aryl-$(C_1-C_6)$-alkoxy bedeutet, wobei Aryl, Aralkyl, Aryloxy und Arylalkoxy jeweils im Arylteil durch einen, zwei oder drei gleiche oder verschiedene Reste aus der Reihe $(C_1-C_4)$-Alkyl, ($C_1$ oder $C_2$)-Alkoxy, Halogen und Nitro, oder ein ($C_1$ oder $C_2$)-Alkylendioxy substituiert sein können; oder

$R^6$ für einen Rest der Formel $-NR^7R^8$ steht;

$R^7$ und $R^8$ gleich oder verschieden sind und Wasserstoff; $(C_1-C_8)$-Alkyl; $(C_4-C_{10})$-Cycloalkyl; $(C_1-C_4)$-Alkyl-$(C_4-C_{10})$-cycloalkyl; $(C_6-C_{12})$-Aryl; $(C_6-C_{12})$-Aryl-$(C_1-C_6)$-alkyl; 2-, 3-oder 4-Pyridyl-$(C_1-C_4)$-alkyl; Amino-$(C_1-C_8)$-alkyl; $(C_1-C_4)$-Alkylamino-$(C_1-C_8)$-alkyl; Di-$(C_1-C_4)$-alkylamino-$(C_1-C_8)$-alkyl; Hydroxy-$(C_1-C_8)$-alkyl oder $(C_1-C_4)$-Alkoxy-$(C_1-C_8)$-alkyl bedeuten, wobei Aryl und Aralkyl jewils im Arylteil durch einen, zwei oder drei gleiche oder verschiedene Reste aus der Reihe $(C_1-C_4)$-Alkyl, ($C_1$ oder $C_2$)-Alkoxy, $(C_1-C_4)$-Alkylamino, Di-$(C_1-C_4)$-alkylamino, Halogen, Hydroxy und Amino, oder ein ($C_1$ oder $C_2$)-Alkylendioxy substituiert sein können; oder

$R^7$ und $R^8$ zusammen mit dem diese tragenden Stickstoffatom einen 4-bis 12-gliedrigen, gesättigten oder

teilweise ungesättigten mono-oder bicyclischen heterocylischen Ring bilden, der als Ringatome 2-11 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe ($C_1$-$C_8$)-Alkyl, Phenyl, Phenyl-($C_1$-$C_6$)-alkyl, Benzoyl, Phenyl-($C_2$-$C_4$)-alkanoyl, 2-oder 3-Furoyl, 2-, 3-oder 4-Pyridyl und 2-, 4-oder 5-Pyrimidinyl substituiert ist, wobei Phenyl, Phenylalkyl, Benzoyl und Phenylalkanoyl ihrerseits jeweils im Phenylteil durch einen, zwei oder drei gleiche oder verschiedene Reste aus der Reihe ($C_1$-$C_4$)-Alkyl, ($C_1$ oder $C_2$)-Alkoxy, Halogen und Nitro, oder ein ($C_1$ oder $C_2$)-Alkylendioxy substituiert sein können;

X Sauerstoff; Imino oder N-($C_1$-$C_8$)-Alkylimino bedeutet;

m = 0, 1, 2, 3, 4 oder 5 ist;

n = 0, 1 oder 2 ist und

s = 0 oder 1 ist ,

sowie deren physiologisch verträgliche Salze, falls solche gebildet werden können.

Alkyl kann geradkettig oder verzweigt sein und ist beispielsweise Methyl, Ethyl, Propyl, Isopropyl, Butyl, Isobutyl, sec.-Butyl, tert.-Butyl, Pentyl, Isopentyl, Neopentyl, sec.-Pentyl, tert.-Pentyl, Hexyl, Isohexyl, Heptyl oder Octyl. Entsprechendes gilt für davon abgeleitete Reste wie Alkoxy, Alkylamino, Dialkylamino, Alkanoyl, Alkoxycarbonyl und Aralkyl.

Aryl ist beispielsweise Phenyl, α-oder β-Naphthyl, 2-, 3-oder 4-Biphenyl; bevorzugt ist Phenyl. Entsprechendes gilt für davon abgeleitete Reste wie Aryloxy, Aralkyl, Aryl, Aroyl und Arylalkanoyl.

Halogen ist Fluor, Chlor, Brom oder Jod; bevorzugt sind Fluor, Chlor und Brom.

Unter $R^4$ in der Bedeutung eines mono-bzw. bicyclischen Heterocyclen-Restes mit 5 bis 7 bzw. 8 bis 10 Ringatomen, wovon 1 bis 9 Ringatome Kohlenstoffatome und 1 bis 2 Ringatome Schwefel-oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Cinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Verbindungen der Formel I besitzen asymmetrische C-Atome. Sowohl die R-als auch die S-Konfigurationen an allen Asymmetriezentren sind Gegenstand der Erfindung. Die Verbindungen der Formel I können daher als optische Isomere, als Diasteromere, als Racemate oder als Gemische derselben vorliegen. Bevorzugt sind jedoch die Verbindungen der Formel I, in denen die mit einem Stern (*) markierten C-Atome S-Konfiguration aufweisen. Falls $R^4$ für die Seitenkette von Cystein steht, wird jedoch die R-Konfiguration dieses Zentrums bevorzugt.

Natürlich vorkommende α-Aminosäuren, wie beispielsweise Ala, Val, Leu, Ile, Phe, Abu, C-Ph-Gly, His, Trp, Lys, Orn, Dab, Dap, Daap, Dapi, Arg, Cit, Glu, Gln, Asp, Asu, Cys, Met, , Thr und Tyr sind z.B. in Ann. Rev. Biochem. 38 [1969] 137-158 und FEBS Letters 64 [1976] 29-35 beschrieben.

Falls $R^4$ für eine geschütze Seitenkette einer natürlich vorkommenden α-Aminosäure steht, wie z.B. geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. z.B. T.W. Greene, "Protective Groups in Organic Synthesis", New York, 1981 oder Bodanszky, Bodanszky, "Principles bzw. Practice of Peptide Synthesis", Berlin, 1984). Im Falle, daß $R^4$ eine geschützte Lysin-Seitenkette bedeutet, werden die bekannten Aminoschutzgruppen, insbesondere aber ($C_1$-$C_6$)-Alkanoyl bevorzugt. Falls $R^4$ eine geschützte Tyrosin-Seitenkette bedeutet, wird die Ether-Schutzgruppe am Sauerstoff, insbesondere ($C_1$-$C_6$)-Alkyl, bevorzugt; besonders bevorzugte Schutzgruppen sind Methyl und Ethyl.

Als Salze kommen insbesondere Alkali-oder Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Bevorzugt sind Verbindungen der Formel I, in welcher

$R^1$ Wasserstoff; ($C_1$-$C_4$)-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy, Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Fluor, Chlor, Brom, Nitro, Hydroxy, Amino, Methylamino und Dimethylamino, oder ein Methylendioxy substituiert ist; oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, drei Methoxy, oder ein Methylendioxy substituiert ist;

$R^3$ Wasserstoff; ($C_1$-$C_4$)-Alkyl; Benzyl; Phenethyl; Cyclopentyl; Cyclohexyl oder Indanyl bedeutet;
$R^4$ Wasserstoff oder die gegebenenfalls geschützte Seitenkette einer natürlich vorkommenden α-Aminosäure bedeutet; oder
$R^3$ und $R^4$ zusammen für einen Rest -$[CH_2]_p$-stehen, der wie oben definiert ist und worin p = 3 oder 4 ist;
$R^5$ Wasserstoff; Methyl; Ethyl; Benzyl oder Phenethyl, welche jeweils im Phenylrest gegebenenfalls durch einen, zwei oder drei gleiche oder verschiedenen Reste aus der Reihe Methoxy, Fluor, Chlor und Brom, oder ein Methylendioxy substituiert sind, bedeutet;
$R^6$ ($C_1$-$C_5$)-Alkoxy, Phenoxy; Phenyl-($C_1$-$C_4$)-alkoxy oder Hydroxy bedeutet, wobei Phenoxy und Phenylalkoxy im Phenylrest jeweils durch einen oder zwei gleiche oder verschiedene Reste aus der Reihe Methyl, Ethyl, Methoxy, Fluor, Chlor und Brom, oder ein Methylendioxy substituiert sein können; oder
$R^6$ für einen Rest der Formel -$NR^7R^8$ steht, worin $R^7$ und $R^8$ gleich oder verschieden sind und Wasserstoff; ($C_1$-$C_6$)-Alkyl; ($C_5$-$C_8$)-Cycloalkyl; ($C_1$ oder $C_2$)-Alkyl-($C_5$-$C_8$)-cycloalkyl; Phenyl; Phenyl-($C_1$-$C_4$)-alkyl; 2-, 3-oder 4-Pyridyl-($C_1$-$C_4$)-alkyl; Amino-($C_1$-$C_4$)-alkyl; ($C_1$ oder $C_2$)-Alkylamino-($C_1$-$C_4$)-alkyl; Di-($C_1$ oder $C_2$)-alkylamino-($C_1$-$C_4$)-alkyl; Hydroxy-($C_1$-$C_4$)-alkyl oder ($C_1$ oder $C_2$)-Alkoxy-($C_1$-$C_4$)-alkyl bedeuten, worin Phenyl und Phenylalkyl jeweils im Phenylteil durch einen oder zwei gleiche oder verschiedene Reste aus der Reihe Methyl, Ethyl, Methoxy, Fluor, Chlor, Brom, Amino, Methylamino und Dimethylamino oder ein Methylendioxy substituiert sein können; oder
$R^7$ und $R^8$ zusammen mit dem diese tragenden Stickstoffatom einen 5-bis 9-gliedrigen heterocyclischen Ring bilden, der als Ringatome 3-8 Kohlenstoffatome und gegebenenfalls ein weiteres Stickstoffatom oder ein Sauerstoffatom enthält und der gegebenenfalls durch ($C_1$-$C_4$)-Alkyl, Phenyl, Phenyl-($C_1$-$C_4$)-alkyl, Benzoyl, Phenyl-($C_2$ oder $C_3$)-alkanoyl, 2-oder 3-Furoyl, 2-, 3-oder 4-Pyridyl oder 2-, 4-oder 5-Pyrimidinyl monosubstituiert ist, wobei Phenyl, Phenylalkyl, Benzoyl und Phenylalkanoyl ihrerseits jeweils im Phenylteil durch einen oder zwei gleiche oder verschiedene Reste aus der Reihe Methyl, Ethyl, Methoxy, Fluor, Chlor und Brom oder ein Methylendioxy substituiert sein können;
X Sauerstoff bedeutet;
m = 0, 1, 2, 3, 4, oder 5 ist;
n = 0 oder 1 ist und
s = 0 oder 1 ist.

Besonders bevorzugt sind Verbindungen der Formel I, in welcher
$R^1$ Wasserstoff; Methyl; Ethyl; Propyl; Isopropyl; Phenyl; o-, m-oder p-Tolyl; o-, m-oder p-Chlorphenyl; o-, m-oder
p-Fluorphenyl; o-, m-oder p-Methoxyphenyl oder 2,3-, 2,4-, 2,5-, 2,6-, 3,4-oder 3,5-Dimethoxyphenyl bedeutet;
$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder
$R^1$ und $R^2$ zusammen für Benzyliden; o-, m-oder p-Methylbenzyliden; o-, m-oder p-Methoxybenzyliden oder 2,3-, 2,4-, 2,5-, 2,6-, 3,4-, oder 3,5-Dimethoxybenzyliden stehen;
$R^3$ Wasserstoff oder Methyl bedeutet;
$R^4$ Wasserstoff; Methyl; n-Propyl; Isopropyl; n-Butyl; Isobutyl; sec.-Butyl oder Benzyl bedeutet; oder
$R^3$ und $R^4$ zusammen für -$[CH_2]_3$-stehen;
$R^5$ Wasserstoff oder Methyl bedeutet;
$R^6$ Methoxy; Ethoxy; Propoxy; Isopropoxy; tert.-Butoxy, n-Butoxy; Benzyloxy; 4-Methoxybenzyloxy; 4-Chlorbenzyloxy; 3,4-Dimethoxybenzyloxy; Phenethyloxy oder einen Rest der Formel -$NR^7R^8$ bedeutet;
$R^7$ und $R^8$ gleich oder verschieden sind und Wasserstoff; ($C_1$-$C_6$)-Alkyl; ($C_5$-$C_8$)-Cycloalkyl; Phenyl; Phenyl-($C_1$-$C_4$)-alkyl; 2-, 3-oder 4-Pyridylmethyl; 2-, 3-oder 4-Pyridylethyl; Amino-($C_1$-$C_4$)-alkyl; Methylamino-($C_1$-$C_4$)-alkyl; Dimethylamino-($C_1$-$C_4$)-alkyl; Hydroxy-($C_1$-$C_4$)-alkyl oder Methoxy-($C_1$-$C_4$)-alkyl bedeuten, wobei Phenyl und Phenylalkyl jeweils im Phenylrest durch einen oder zwei gleiche oder verschiedene Reste aus der Reihe Methyl, Methoxy, Fluor, Chlor, Brom, Amino, Methylamino und Dimethylamino, oder ein Methylendioxy substituiert sein können;
oder $R^7$ und $R^8$ zusammen mit dem diese tragenden Stickstoffatom für Pyrrolidino, Piperidino, Tetrahydropyridino, Morpholino, Azepino, N,N-Heptamethylenimino, Piperazino oder Homopiperazino stehen, wobei die genannten Heterocyclenreste durch ($C_1$-$C_4$)-Alkyl, Phenyl, Phenyl-($C_1$-$C_4$)-alkyl, Benzoyl, Phenyl-($C_2$ oder $C_3$)-alkanoyl oder 2-, 3-oder 4-Pyridyl monosubstituiert sein können und worin Phenyl, Phenylalkyl, Benzoyl und Phenylalkanoyl ihrerseits im Phenylteil durch einen oder zwei gleiche oder verschiedene Reste aus der

Reihe Methyl, Methoxy, Fluor, Chlor oder Brom substituiert sein können;
X Sauerstoff bedeutet;
m = 0, 1, 2, 3, 4 oder 5 ist;
n = 0 oder 1 ist und
s = 0 oder 1 ist.

Insbesondere bevorzugt sind solche Verbindungen der Formel I, in welcher
$R^2$ Wasserstoff bedeutet;
$R^3$ und $R^4$ zusammen für $-[CH_2]_3-$stehen;
$R^5$ Wasserstoff bedeutet;
X Sauerstoff bedeutet;
m = 1, 2, 3 oder 4 ist;
n = 1 ist und
s = 0 oder 1 ist;
sowie solche Verbindungen der Formel I, in welcher
$R^5$ Wasserstoff bedeutet;
X Sauerstoff bedeutet;
m = 1, 2, 3 oder 4 ist;
n = 0 ist und
s = 0 oder 1 ist.

Die Erfindung betrifft auch ein Verfahren zur Herstellung von Verbindungen der Formel I, das dadurch gekennzeichnet ist, daß man eine Verbindung der Formel IV

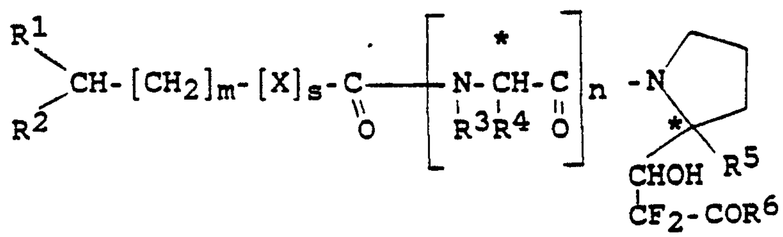

(IV)

in welcher $R^1$, $R^2$, $R^3$, $R^4$ $R^5$ und $R^6$, X, m, n, und s die gleiche Bedeutung wie in Formel I haben, oxidiert und die so erhaltene Verbindung gegebenenfalls in ihre physiologisch verträglichen Salze überführt.

Dabei kommen folgende Oxidationsmittel in Betracht; Mangandioxid, Natrium-oder Kaliumdichromat; Jones Reagenz ($CrO_3$ in wäßriger Schwefelsäure), N-Bromacetamid, N-Bromsuccinimid, Dimethylsulfoxid, Cerammonnitrat, $CrO_3$ in Pyridin, tert.-Butylchromat, Dipyridin-$CrO_3$, Kaliumhypochlorit oder Jodosobenzol. Als Reaktionsmedium sind Petrolether, Benzol, Tetrachlorkohlenstoff oder, im Falle von $MnO_2$, verdünnte Schwefelsäure geeignet. Die Oxidation wird zwischen 0 °C und dem Siedepunkt des Reaktionsgemisches durchgeführt. Bevorzugt ist die Oxidation mit Dimethylsulfoxid mit verschiedenen Zusätzen, wie sie beispielsweise in Houben-Weyl, Band E 3, Seiten 275-281 beschrieben wird. Insbesondere bevorzugt sind die Dimethylsulfoxid-Oxidation in Gegenwart von Oxalylchlorid sowie das in J. Org. Chem. 48 [1983] 4155 beschriebene Verfahren.

Verbindungen der Formel IV stellt man beispielsweise her, indem man eine Verbindung der Formel II

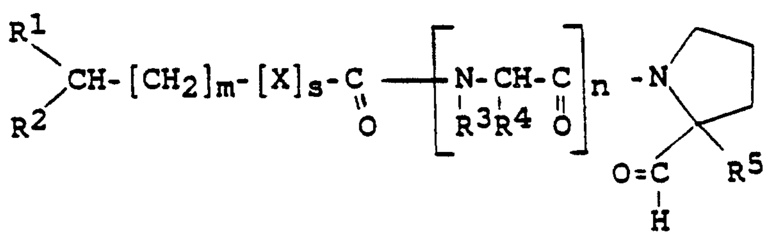

(II)

in welcher $R^1$, $R^2$, $R^3$, $R^4$, $R^5$, X, m, n und s die gleiche Bedeutung wie in Formel I haben, mit einer Verbindung der Formel III

$Y-CF_2-COR^6$ (III)

in welcher $R^6$ die gleiche Bedeutung wie in Formel I hat und Y Halogen, vorzugsweise Chlor, Brom oder

Jod bedeutet, in einem inerten Lösungsmittel wie einem Ether, Dimethylformamid, Diethylformamid, N-Methylpyrrolidon oder Dimethylsulfoxid, insbesondere in Tetrahydrofuran, unter Zuhilfenahme eines Metalls wie Lithium, Natrium, Kalium, Magnesium oder Zink, wobei das letzere bevorzugt ist, bei 0 °C bis zum Siedepunkt des Lösungsmittels, vorzugsweise bei 40 bis 100 °C mit oder ohne zusätzliche Behandlung mit Ultraschall umsetzt.

Verbindungen der Formel IV, worin $R^6$ den Rest $-NR^7R^8$ bedeutet und $R^1$, $R^2$, $R^3$, $R^4$, $R^5$, X, m, n, und s wie in Formel I definiert sind, erhält man aus entsprechenden Verbindungen der Formel IV, worin $R^6$ Hydroxy bedeutet, durch Umsetzung mit Verbindungen der Formel V

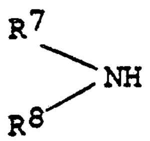 (V)

in welcher $R^7$ und $R^8$ wie in Formel I definiert sind. Man verfährt vorzugsweise in Analogie zu den in der Peptidchemie gebräuchlichen Amidknüpfungsverfahren, wie sie beispielsweise in Houben-Weyl, Band 15/2, Seite 1-364, in Bodanszky, "Principles bzw. Practice in Peptide Synthesis", Berlin, 1984 und den US-Patenten 4,311,592 und 4,426,325 beschrieben werden, indem man die Umsetzung in einem organischen Lösungsmittel wie DMF, $CH_2Cl_2$, DMA in Gegenwart von Kupplungshilfsmitteln, wie Carbodiimiden (z.B. Dicyclohexylcarbodiimid), Diphenylphosphorylazid, Alkanphosphoräureanhydriden, Dialkylphosphinsäureanhydriden oder N,N-Succinimidylcarbonaten in einem Lösungsmittel wie $CH_3CN$ durchgeführt. Die Verbindungen der Formel IV können in Aktivester (z.B. mit 1-Hydroxybenzotriazol), gemischte Anhydride (z.B. mit Chlorameisensäureestern), Azide oder Carbodiimid-Derivate überführt und damit aktiviert werden (vgl. Schröder, Lübke, The Peptides, Band 1, New York 1965, Seiten 76-136). Die Reaktion wird vorzugsweise zwischen -20 °C und dem Siedepunkt des Reaktionsgemisches durchgeführt.

Weiterhin kann man zur Herstellung von Verbindungen der Formel IV, worin $R^6$ für $-NR^7R^8$ steht und die übrigen Reste und Variablen wie in Formel I definiert sind, entsprechende Verbindungen der Formel IV, worin $R^6$ für $(C_1-C_6)$-Alkoxy-Phenoxy oder $(C_7-C_{13})$-Aralkyloxy steht, mit Verbindungen der oben definierten Formel V in einem geeigneten organischen Lösungsmittel wie einem niederen Alkohol, Dimethylformamid oder Dimethylsulfoxid, vorzugsweise in Ethanol, bei 20 °C bis zum Siedepunkt des Reaktionsgemisches vorzugsweise bei 40 - 80 °C umsetzen.

Verbindungen der Formel II sind entweder aus der Literatur bekannt oder können in Analogie zu literaturbekannten Verfahren hergestellt werden; einige Vertreter sind beispielsweise in den europäischen Patentanmeldungen EP-A-172 458 und EP-A-201 742 und der japanischen Patentanmeldung 1183-297 sowie in Life Sci. 33, 2149 (1983) beschrieben.

Die erfindungsgemäßen Verbindungen der Formel I sind Hemmstoffe der Prolyl-Endopeptidase (EC 3.4.21.26). Von diesem Enzym ist bekannt, daß es Neuropeptide wie Substanz P, Neurotensin, LHRH, TRH, Vasopressin sowie Angiotensin II abbaut (Life Sci. 33, 2149 (1983)). Diese Neuropeptide sind mit wichtigen Funktionen im Zentralnervensystem (ZNS) assoziiert. Durch Hemmung ihres Abbaus mittels Hemmung der Propyl-Endopeptidase werden durch Verbindungen der Formel I verschiedenartige Wirkungen im ZNS ausgelöst, insbesondere anti-amnestische, antipsychotische, anxiolytische und antidepressive Wirkungen.

Verbindungen der Formel I eignen sich daher zur Behandlung verschiedener Erkrankungen des Zentralnervensystems, insbesondere als Nootropika und Antipsychotika bei Warmblütern, vorzugsweise beim Menschen. Die Anwendung der erfindungsgemäßen Verbindungen kann intravenös, subcutan oder peroral für sich allein oder in Kombination mit anderen ZNS-wirksamen Substanzen erfolgen.

Die Dosierung liegt je nach Art und Schwere der zu behandelnden Erkrankung bei 0,0001-10 mg/kg/Tag, insbesondere bei 0,001-1 mg/kg/Tag. Sie kann in schweren Fällen auch erhöht werden, da toxische Eigenschaften bisher nicht beobachtet wurden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche

und tierischen Öle in Betracht, wie Sonnenblumenöl oder Lebertran.

Zur subcutanen oder intravenösen Applikation werden die aktiven Verbindungen oder deren physiologisch verträgliche Salze, gewünschten falls mit den dafür üblichen Substanzen wie Lösungsvermittler, Emulgatoren, oder weiteren Hilfsstoffen in Lösung, Suspension oder Emulsion gebracht. Als Lösungsmittel für die jeuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose-oder Mannitlösungen, oder auch eine Mischung aus den verschiedenen genannten Lösungsmitteln.

Die folgenden Beispiele sollen die vorliegende Erfindung erläutern, ohne daß diese darauf beschränkt wäre.

Beispiel 1

3-[N-(N-Benzyloxycarbonyl-S-prolyl)-pyrrolidin-2-S-yl]-3-oxo-2,2-difluorpropionsäuremethylester

a) 3-[N-(N-Benzyloxycarbonyl-S-prolyl)-pyrolidin-2-S-yl]-3-hydroxy-2,2-difluorpropionsäuremethylester

1,2 g Zinkpulver werden mit 3,75 g Brom-difluoressigsäuremethylester in 30 ml Tetrahydrofuran zum Sieden erhitzt. Anschließend gibt man 5 g N-Benzyloxycarbonyl-S-prolyl-S-prolinal zu und erhitzt noch 15 Minuten. Nach Abkühlen wird mit 0,5 N Salzsäure/Essigester aufgearbeitet.

Chromatographie an Kieselgel mit Essigester/Cyclohexan (3:1) als Laufmittel ergibt 5,1 g der Titelverbindung,
Schmp. 119-120 °C,$[\alpha]_D^{22}$ = -53,9 ° (c = 0,232, $CH_2Cl_2$).

b) 3-[N-(N-Benzyloxycarbonyl-S-prolyl)-pyrrolidin-2-S-yl]-3-oxo-2,2-difluorpropionsäuremethylester

Zu 70 µl Oxalylchlorid in 10 ml Dichlormethan gibt man bei -78 °C 0,12 ml Dimethylsulfoxid. Nach 2 Minuten gibt man 0,32 g der Verbindung aus Beispiel 1a) zu, nach weiteren 5 Minuten 0,78 ml Triethylamin. Nach Aufwärmen auf Raumtemperatur wird mit 0,1 N Salzsäure, wäßriger 1 M Natriumhydrogencarbonatlösung und gesättigter wäßriger Kochsalzlösung gewaschen, mit $Na_2SO_4$ getrocknet und eingeengt.

Nach Chromatographie an Kieselgel mit Essigester/Cyclohexan (3:1) erhält man 0,17 g der Titelverbindung als Öl.
$^1$H-NMR ($CDCl_3$),
$\delta$ = 7,4-7,2 (m, 5H); 5,2-4,7 (m,3H); 4,55-4,3 (m, 1H); 3,92 3,88 (2s, 3H); 3,95-3,8 (m 1H) 3,7-3,3 (m 4H); 2,3-1,5 (m, 7H) ppm.

Beispiel 2

3-[N-(N-Benzyloxycarbonyl-S-prolyl)-pyrrolidin-2-S-yl]-3-oxo-2,2-difluorpropionsäure-N-benzylamid

a) 3-[N-(N-Benzyloxycarbonyl-S-prolyl)-pyrrolidin-2-S-yl]-3-hydroxy-2,2-difluorpropionsäure-N-benzylamid

0,308 g der Verbindung aus Beispiel 1a) werden mit 0,3 ml Benzylamin in 10 ml absolutem Ethanol unter Stickstoff 4 Stunden am Rückfluß erhitzt. Nach Einengen wird am Kieselgel mit Essigester/Cyclohexan (3:1) chromatographiert. Man erhält 0,25 g der Titelverbindung, Schmp. 135 °C

b) 3-[N-(N-Benzyloxycarbonyl-S-prolyl)-pyrrolidin-2-S-yl]-3-oxo-2,2-difluorpropionsäure-N-benzylamid

Die Verbindung aus Beispiel 2a (0,93 g) wird mit 0,19 ml Oxalylchlorid und 0,3 ml DMSO in 5 ml Dichlormethan analog dem in Beispiel 1 b) angegebenen Verfahren oxidiert. Nach Chromatographie an Kieselgel mit Essigester/Cyclohexan (3:1) erhält man 0,335 g der Titelverbindung als Öl.
$^1$H-NMR ($CDCl_3$):
$\delta$ = 7,6 (s,1H); 7,4-7,2 (m,10H); 5,2-4,9 (m,3H); 4,7-4,3 (m,4H); 3,95-3,5 (m,1H); 3,5-3,3 (m,3H); 2,4-1,7 (m,7H) ppm.

Beispiel 3

3-[N-[4-(4-Methoxyphenyl)-butyryl]-pyrrolidin-2-S-yl]-3-oxo-2,2-difluorpropionsäuremethylester

a) 3-[N-[4-(4-Methoxy-phenyl)-butyryl]-pyrrolidin-2-S-yl]-3-hydroxy-2,2-difluorpropionsäuremethylester

Analog dem in Beispiel 1a) angegebenen Verfahren erhält man aus 5,4 g Brom-difluoressigsäuremethylester und 6 g 4-(4-Methoxyphenyl-butyryl)-S-prolinal mit 1,7 g Zinkpulver nach Chromatographie an Kieselgel (Essigester/Cyclohexan 2:1) 6,2 g der Titelverbindung als Öl.
$^1$H-NMR ($CDCl_3$):
$\delta$ = 7,2-6,7 (AB-System,4H); 4,7-4,2 (m,1H); 4,0-3,5 (m,1H); 3,9 + 3,8 (2s,3H); 3,6-3,1 (m,2H); 2,6 (t,2H); 2,4-1,6 (m,8H) ppm.

b) 3-[N-[4-(4-Methoxyphenyl)butyryl]-pyrrolidin-2-S-yl]-3-oxo-2,2-difluorpropionsäuremethylester

Analog der in Beispiel 1 b) angegebenen Vorschrift erhält man aus 0,8 g der Verbindung aus Beispiel 3 a) 0,54 g der Titelverbindung als Öl; $[\alpha]_D^{23}$ = +55,3° (c = 0,66, ($CH_2Cl_2$)
$^1$H-NMR ($CDCl_3$:
$\delta$ = 7,2-6,6 (AB-System,4H); 4,92 (t,1H); 3,97 + 3,8 (2s,3H); 3,6-3,3 (m,2H); 2,6 (t,2H); 2,4-1,7 (m,8H) ppm.

Analog der in Beispiel 2 a) angegebenen Vorschrift wurden aus 3-[N-[4-(4-Methoxyphenyl)-butyryl]-pyrrolidin-2-S-yl]-3-hydroxy-2,2-difluorpropionsäuremethylester und den entsprechenden Aminen hergestellt:

Beispiel 4

3-[N-[4-(4-Methoxyphenyl)-butyryl-pyrrolidin-2-S-yl]-3-hydroxy-2,2-difluorpropionsäure-N-benzylamid

Es wurden 2 Isomere im Verhältnis 8,5:1 erhalten, die durch Chromatographie an $SiO_2$ (Laufmittel Essigester/Cyclohexan = 1:1) getrennt wurden.

Isomer 1: Öl, $[\alpha]_D^{23}$ = -34° (c = 0,3, $CH_2Cl_2$)
$^1$H-NMR ($CDCl_3$):
$\delta$ = 7,3 (s,5H); 7,2-6,6 (AB-System,4H); 4,6-4,4 (m,3H); 4,3-3,3 (m,3H); 3,8 (s,3H); 2,63 (t,2H); 2,4-1,8 (m,8H) ppm.

Isomer 2: Öl, $[\alpha]_D^{23}$ = -23,4° (c = 0,124, $CH_2Cl_2$)
$^1$H-NMR($CDCl_3$):
δ = 7,3 (s,5H); 7,2-6,6 (AB-System, 4H); 4,6-4,2 (m,3H); 3,8 (s,3H); 3,6-3,0 (m,3H); 2,63 (t,2H); 2,5-1,7 (m,8H) ppm.

Beispiel 5

3-[N-[4-(4-Methoxyphenyl)-butyryl]-pyrrolidin-2-S-yl]-3-hydroxy-2,2-difluorpropionsäure-N-(2-phenyl-ethyl)-amid

Es wurden 2 Isomere im Verhältnis 7,8:1 erhalten, die durch Chromatographie an $SiO_2$ (Laufmittel: Essigester/Cyclohexan = 1:1) getrennt wurden.

Isomer 1 : Öl, $[\alpha]_D^{23}$ = -40,9° (c = 0,2, $CH_2Cl_2$)
$^1$H-NMR ($CDCl_3$):
δ = 7,2 (s,5H); 7,2-6,7 (AB-System, 4H); 6,5 (s,1H); 4,6-4,3 (m,1H); 4,2-3,2 (m,5H); 3,8 (s,3H); 3,0-1,6 (m,12H) ppm.

Isomer 2: Öl, $[\alpha]_D^{23}$ = -22,6° (c = 0,112, $CH_2Cl_2$)
$^1$H-NMR ($CDCl_3$):
δ = 7,2 (s,5H); 7,2-6,7 (AB-System,4H); 4,6-4,0 (m,2H); 3,8 (s,3H); 3,7-3,1 (m,4H); 2,82 (t,H); 2,6 (t,2H); 2,4-1,7 (m,10H) ppm.

Beispiel 6

3-[N-[4-(4-Methoxyphenyl)-butyryl]-pyrrolidin-2-S-yl]-3-hydroxy-2,2-difluorpropionsäure-N-(3-dimethylaminopropyl)-amid

Öl
$^1$H-NMR ($CDCl_3$):
δ = 8,4 (S,1H); 7,2-6,6 (AB-System,4H); 4,7-4,4 (m,1H); 4,3-3,6 (m,1H); 3,8 (s,3H); 3,6-3,3 (m,6H); 2,8-1,5 (m,12H); 2,25 (s,6H) ppm.

Beispiel 7

3-[N-[4-(4-Methoxyphenyl)-butyryl]-pyrrolidin-2-S-yl]-3-hydroxy-2,2-difluorpropionsäure-pyrrolidid

Öl
$^1$H-NMR ($CDCl_3$):
δ = 7,2-6,6 (AB-System,4H); 5,9 (dd,1H); 4,7-4,4 (m,1H); 4,2-3,2 (m,7H); 3,8 (s,3H); 2,6 (t, 2H); 2,4-1,5 (m,12H) ppm.

Beispiel 8

3-[N-[4-(4-Methoxyphenyl)-butyryl]-pyrrolidin-2-S-yl]-3-hydroxy-2,2-difluorpropionsäure-N-(2-picolyl)-amid

Es bildeten sich 2 Isomere (Verhältnis ca. 8:1), das weniger polare Isomer wurde durch Säulenchromatographie an sich (Laufmittel Essigester) abgetrennt, Öl.
$[\alpha]_D^{22}$ = -35,5° (c = 0,29, $CH_2Cl_2$)
$^1$H-NMR ($CDCl_3$:
δ = 8,57 (d,1H); 7,7 (dt,2H); 7,32 (d,1H); 7,3-7,2 (m,1H); 7,1 (d,2H); 6,83 (d,2H); 6,0 (s,1H); 4,8-4,5 (m,3H); 4,05-3,9 (m,1H); 3,8 (s,3H); 3,4 (m,2H); 2,6 (t,2H); 2,4 (t,2H); 2,2-1,85 (m,6H) ppm.

Beispiel 9

3-[N-[4-(4-Methoxyphenyl)-butyryl]-pyrrolidin-2-S-yl]-2,2-difluor-3-oxopropionsäure-N-benzylamid

0,78 g der Verbindung aus Beispiel 4 (beide Isomere) werden nach dem in Beispiel 1 b) angegebenen Verfahren oxidiert. Man erhält 0,7 g der Titelverbindung als Öl.
$[\alpha]_D^{22}$ = +8,3° (c = 0,444, $CH_2Cl_2$)
$^1$H-NMR ($CDCl_3$):
δ = 7,6 (s,1H); 7,3 (s,1H); 7,2-6,7 (AB-System,4H); 4,98 (t,3H); 4,6-4,3 (AB-System,2H); 3,8 (s,3H); 3,6-3,3 (m,2H); 2,55 (t,3H); 2,4-1,7 (m,8H) ppm.

Beispiel 10

3-[N-[4-[4-Methoxyphenyl)-butyryl]-pyrrolidin-2-S-yl]-2,2-difluor-3-oxopropionsäure-N-(2-phenyl-ethyl)-amid

0,83 g der Verbindung aus Beispiel 5 (beide Isomere) werden nach dem in Beispiel 1 b) angegebenen Verfahren oxidiert. Man erhält 0,78 g der Titelverbindung als Öl
$[\alpha]_D^{23}$ = +16,7° (C = 0,86, $CH_2Cl_2$)
$^1$H-NMR ($CDCl_3$):
δ = 7,2 (s,5H); 7,2-6,7 (AB-System,4H); 4,9 (t,1H); 3,8 (s,3H); 3,6-3,3 (m,4H); 2,86 (t,2H); 2,6 (t,2H); 2,4-1,3 (m, 10H) ppm.

Beispiel 11

3-[N-[4-(4-Methoxyphenyl)-butyryl]-pyrrolidin-2-S-yl]-2,2-difluor-3-oxo-propionsäure-N-(3-dimethylaminopropyl)-amid

0,66 g der Verbindung aus Beispiel 6 werden analog dem in Beispiel 1 b) beschriebenen Verfahren oxidiert. Man erhält 0,19 g der Titelverbindung als Öl nach Chromatographie an $SiO_2$ mit $CH_2Cl_2/CH_3OH$ (8:2).
$^1$H-NMR ($CDCl_3$):
δ = 9,0 (s,1H); 7,1 (d,2H); 6,83 (d,2H); 5,0 (dd,1H); 3,8 (s,3H); 3,6-3,4 (m,3H); 2,6 (t,2H); 2,4 (t,2H); 2,33 (s,6H); 2,1-1,6 (m,8H) ppm.

Beispiel 12

3-[N-[4-(4-Methoxyphenyl)-butyryl]-pyrrolidin-2-S-yl]-2,2-difluor-3-oxo-propionsäure-pyrrolidid

0,46 g der Verbindung aus Beispiel 7 werden analog dem in Beispiel 1 b) beschriebenen Verfahren oxidiert. Man erhält 0,35 g der Titelverbindung als Kristalle vom Schmp. 94-95 °C.
$[\alpha]_D^{22}$ = +41,4° (c = 0,294, $CH_2Cl_2$)

Beispiel 13

3-[N-[4-(4-Methoxyphenyl)-butyryl]-pyrrolidin-2-S-yl]-2,2-difluor-3-oxo-propionsäure-N-(2-picolyl)-amid

0,81 g der Verbindung aus Beispiel 8 werden analog dem in Beispiel 1 b) beschriebenen Verfahren oxidiert. Man erhält 0,8 g der Titelverbindung als Öl.
$[\alpha]_D^{22}$ = +10° (c = 0,412, $CH_2Cl_2$)
$^1$H-NMR ($CDCl_3$):
δ= 8,55 (m,1H); 8,3-8,0 (m-1H); 7,7-7,1 (m,2H); 7,2-6,3 (AB-System,4H); 6,05 (t,1H); 4,65 (AB-System,2H); 3,8 (s,3H); 3,5 (t,2H); 2,55 (t,2H); 2,5-1,6 (m,8H) ppm.

Beispiel 14

3-[N-(3,3-Diphenylpropionyl)-pyrrolidin-2-S-yl]-2,2-difluor-3-oxo-propionsäuremethylester

a) 3-[N-(3,3-Diphenylpropionyl)-pyrrolidin-2-S-yl]-2,2-difluor-3-hydroxypropionsäuremethylester

Analog dem in Beispiel 1 a) beschriebenen Verfahren werden 6,14 g N-(3,3-Diphenylpropionyl)-S-prolinal mit 4,95 g Methylbromdifluoracetat und 1,55 g Zink umgesetzt. Nach Säulenchromatographie an $SiO_2$ mit Essigester/Cyclohexan (1:1) als Laufmittel erhält man 6,45 g der Titelverbindung als Öl.
$^1$H-NMR ($CDCl_3$:
δ= 7,2 (s,10H); 5,9 (dd,1H); 4,6 (t,1H); 4,6-4,2 (m,1H); 4,1-3,5 (m,1H); 3,87 (s,3H); 3,25 (t,2H); 3,0 (d,2H); 2,0-1,6 (m,4H) ppm.

b) 3-[N-(3,3-Diphenylpropionyl)-pyrrolidin-2-S-yl]-2,2-difluor-3-oxopropionsäuremethylester

Analog dem in Beispiel 1 b) beschriebenen Verfahren werden 0,8 g der Verbindung aus a) oxidiert. Man erhält 0,65 g der Titelverbindung als Öl.
$[\alpha]_D^{23}$ = +30,5° (c = 0,57, $CH_2Cl_2$)
$^1$H-NMR ($CDCl_3$):
δ= 7,2 (s,10H); 4,83 (t,1H); 4,63 (t,1H); 3,83 (s,3H); 3,6-3,2 (m,2H); 3,0 (d,2H); 2,3-1,8 (m,6H) ppm.

Analog der in Beispiel 2 a) angegebenen Vorschrift wurden aus jeweils 0,8 g 3-[N-(3,3-diphenylpropionyl)-pyrrolidin-2-S-yl]-2,2-difluor-3-hydroxy-propionsäure-methylester und 4 Äquivalenten des jeweiligen Amins hergestellt:

Beispiel 15

3-[N-(3,3-Diphenylpropionyl)-pyrrolidin-2-S-yl]-2,2-difluor-3-hydroxypropionsäure-N-benzylamid

Öl: 0,81 g
$^1$H-NMR ($CDCl_3$):
δ = 7,3 (s,5H); 7,2 (s,10H); 5,95 (dd,1H); 4,8-4,3 (m,3H); 4,3-3,6 (m,1H); 3,4 (t,2H); 3,05 (d,2H); 2,2-1,5 (m,4H) ppm.

Beispiel 16

3-[N-(3,3-Diphenylpropionyl)-pyrrolidin-2-S-yl]-2,2-difluor-3-hydroxypropionsäure-N-(2-picolyl)-amid

Öl: 0,88 g
$^1$H-NMR ($CDCl_3$):
δ = 8,55 (d,1H); 7,73-7,65 (m,2H); 7,35-7,15 (m,11H); 4,7-4,3 (m,4H); 3,9-3,8 (ddd,1H); 3,3 (t,2H); 3,15-3,0 (m,2H); 2,0-1,7 (m,4H) ppm.

Beispiel 17

3-[N-(3,3-Diphenylpropionyl)-pyrrolidin-2-S-yl]-2,2-difluor-3-oxopropionsäure-N-benzyl-amid

0,81 g der Verbindung aus Beispiel 15 werden nach dem in Beispiel 1 b) beschriebenen Verfahren oxidiert. Man erhält 0,67 g der Titelverbindung als Öl.
$[\alpha]_D^{22}$ = -3,5° (c = 0,36, $CH_2Cl_2$)
$^1$H-NMR ($CDCl_3$):
δ = 7,4-7,0 (m,15H); 5,0-4,5 (m,1H); 4,5 (AB-System,2H); 3,6-3,2 (m,2H); 3,0 (d,2H); 2,4-1,6 (m,4H) ppm.

Beispiel 18

3-[N-(3,3-Diphenylpropionyl)-pyrrolidin-2-S-yl]-2,2-difluor-3-oxopropionsäure-N-(2-picolyl)-amid

0,88 g der Verbindung aus Beispiel 1 b) werden nach dem in Beispiel 1 b) beschriebenen Verfahren oxidiert. Man erhält 0,58 g der Titelverbindung als Öl.
$[\alpha]_D^{22}$ = -4,4° (c = 9,476, $CH_2Cl_2$)
$^1$H-NMR ($CDCl_3$):
δ = 8,55 (d,1H); 8,3-7,6 (m,2H); 7,5-7,0 (m,11H); 5,1-4,5 (m,5H); 3,6-3,3 (m,2H); 3,0 (d,2H); 2,4-1,7 (m,4H) ppm.

Beispiel 20

3-[N-[N-(4-Phenyl-butyryl)-S-prolyl]-pyrrolidin-2-S-yl]-3-oxo-2,2-difluorpropionsäuremethylester

a) 3-[N-[N-(4-Phenyl-butyryl)-S-prolyl]-pyrrolidin-2-S-yl]-3-hydroxy-2,2-difluorpropionsäuremethylester

Analog dem in Beispiel 1 a) beschriebenen Verfahren werden 2,3 g [N-[N-(4-Phenyl-butyryl)-S-prolyl]-S-prolinal mit 0,5 g Zink und 1,4 g Bromdifluoressigsäuremethylester umgesetzt. Man erhält 1,65 g der Titelverbindung nach Chromatographie an $SiO_2$ mit Essigester/Methanol (40:1) als Laufmittel als Öl.
$^1$H-NMR ($CDCl_3$):
δ= 7,3-7,15 (m,5H); 5,0-4,2 (m,2H); 4,2-3,9 (m,2H); 3,9 (s,3H); 3,8-2,9 (m,3H); 2,7 (m,2H); 2,4-1,8 (m,12H) ppm.

b) 3-[N-[N-(4-Phenyl-butyryl)-S-prolyl]-pyrrolidin-2-S-yl]-3-oxo-2,2-difluorpropionsäuremethylester

Analog dem in Beispiel 1 b) angegebenen Verfahren werden 0,55 g der Verbindung aus a) oxidiert. Nach Chromatographie ($SiO_2$, Essigester) erhält man 0,26 g der Titelverbindung als Öl.
$[\alpha]_D^{22}$ = -21,7° (c = 2,2, $CH_2Cl_2$)
$^1$H-NMR ($CDCl_3$):
δ= 7,3-7,1 (m,5H); 5,1-4,9 (m,1H); 4,75-4,6 (m,1H); 4,0-3,8 (m,1H); 3,9 s,3H); 3,7-3,3 (m,4H); 2,7 (t,2H); 2,9-1,8 (m,12H); ppm.

Beispiel 21

3-[N-[N-(4-Phenylbutyryl)-S-prolyl]-pyrrolidin-2-S-yl]-3-oxo-2,2-difluor-propionsäure-N-benzylamid

a) 3-[N-[N-(4-Phenylbutyryl)-S-prolyl]-pyrrolidin-2-S-yl]-3-hydroxy-2,2-difluor-propionsäure-N-benzylamid

Analog dem in Beispiel 2 a) beschriebenen Verfahren werden 0,55 g der Verbindung aus Beispiel 20 a) mit 0,6 ml Benzylamin umgesetzt. Man erhält 0,5 g der Titelverbindung als Öl.
$^1$H-NMR ($CDCl_3$):
δ= 7,4-7,1 (m,10H); 6,9-6,7 (m,1H); 4,9-4,3 (m,4H); 4,1-3,8 (m,2H); 3,7-2,9 (m,3H); 2,7(t,2H); 2,4-1,8 (m,12H); ppm.

b) 3-[N-[N-(4-Phenylbutyryl)-S-prolyl]-pyrrolidin-2-S-yl]-3-oxo-2,2-difluor-propionsäure-N-benzylamid

Nach dem in Beispiel 1 b) angegebenen Verfahren werden 0,5 g der Verbindung aus a) oxidiert. Man erhält 0,38 g der Titelverbindung als Öl.
$[\alpha]_D^{22}$ = -29,3° (c = 3,1, $CH_2Cl_2$)
$^1$H-NMR ($CDCl_3$):
δ= 7,7 (s,1H); 7,4-7,1 (m,10H); 5,05 (dd,1H); 4,65-4,3 (m,3H); 3,95 (m,1H); 3,6 (m,1H); 3,5-3,3 (m,2H); 2,67 (t,2H); 2,4-1,8 (m,12H); ppm.

Beispiel 22

3-[N-[N-(3,3-Diphenylpropionyl)-S-prolyl]-pyrrolidin-2-S-yl]-3-oxo-2,2-difluor-propionsäuremethylester

a) 3-[N-[N-(3,3-Diphenylpropionyl)-S-prolyl]-pyrrolidin-2-S-yl]-3-hydroxy-2,2-difluorpropionsäuremethylester

Analog dem in Beispiel 1 a) beschriebenen Verfahren werden 17,5 g N-[N-(3,3-Diphenylpropionyl)-S-prolyl]-S-prolinal mit 3,4 g Zink und 10,7 g Methylbromdifluoracetat umgesetzt. Man erhält 10,5 g der Titelverbindung als Öl.
$^1$H-NMR ($CDCl_3$):
δ = 7,3 (s,10H); 4,8-3,2 (m,7H); 3,9 (s,3H); 3,05 (d,2H); 2,4-1,6 (m,8H) ppm.

b) 3-[N-[N-(3,3-Diphenylpropionyl)-S-prolyl]-pyrrolidin-2-S-yl]-3-oxo-2,2-difluor-propionsäuremethylester

Analog dem in Beispiel 1 b) angegebenen Verfahren werden 0,6 g der Verbindung aus a) oxidiert. Nach Chromatographie ($SiO_2$, Essigester/Cyclohexan 2:1) erhält man 0,31 g der Titelverbindung als Öl.
$[\alpha]_D^{24}$ = -33,8° (c = 0,408, $CH_2Cl_2$)
$^1$H-NMR ($CDCl_3$):
δ = 7,3-7,1 (m,10H); 5,0 (dd,1H); 4,65 (dd,1H); 4,55 (dd,1H); 3,9 (m,4H); 3,3 (m,1H); 3,1 (m,2H); 3,05 (ddd,2H); 2,3-1,7 (m,8H) ppm.

Analog den in Beispiel 2 angegebenen Verfahrensschritten erhielt man unter Verwendung geeigneter Ausgangsmaterialien die in der folgenden Tabelle aufgeführten Verbindungen der Formel I a

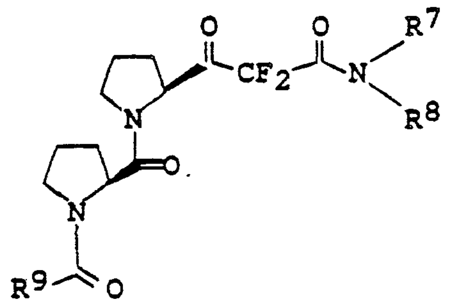

I a

| Bsp. Nr. | $R^7$ | $R^8$ | $R^9$ | $[\alpha]_D^{24}$ ($CH_2Cl_2$) | $^1$H-NMR ($CDCl_3$); δ-Werte (ppm) |
|---|---|---|---|---|---|
| 23 | H | $-(CH_2)_2-C_6H_5$ | $C_6H_5-CH_2-O-$ | -36,7° (c = 0,572) | 7,38 (s,5H); 5,2 (s,2H); 5,1-3,3 (m,8H); 2,8 (t,2H); 2,4-1,5 (m,8H). |

| Bsp. Nr. | $R^7$ | $R^8$ | $R^9$ | $[\alpha]_D^{24}(CH_2Cl_2)$ | $^1$H-NMR ($CDCl_3$); δ-Werte (ppm) |
|---|---|---|---|---|---|
| 24 | H | $-(CH_2)_3-C_6H_5$ | $C_6H_5-CH_2-O-$ | -33,6° (c = 0,438) | 7,3 (s,5H); 5,2 (s,2H); 5,1-3,2 (m,8H); 2,6 (t,2H); 2,4-1,6 (m,10H). |
| 25 | H | $-CH_2-$ 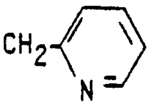 | $C_6H_5-CH_2-O-$ | -44,3° (c = 0,56) | 8,55 (d,1H); 8,02 (s,1H); 7,6 (q,1H); 7,4-7,15 (m,6H); 5,2-4,3 (m,8H); 3,7-3,3 (m,4H); 2,4-1,7 (m,8H). |
| 26 | H | 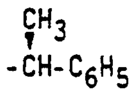 | $C_6H_5-CH_2-O-$ | -7,6° (c = 0,434) | 7,7-7,5 (2d,1H); 7,4-7,2 (m,5H); 5,2-4,9 (m,2H); 5,1-4,35 (m,2H); 3,95-3,55 (m,1H); 3,6-3,3 (m,4H); 2,4-1,7 (m,8H); 1,55 (2d,3H). |
| 27 | H | 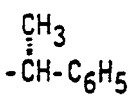 | $C_6H_5-CH_2-O-$ | -58° (c = 0,512) | 7,7 (m,1H); 7,4-7,2 (m,5H); 5,2-4,9 (m,2H); 5,1-4,6 (m,2H); 3,9-3,3 (m,5H); 2,3-1,8 (m,8H); 1,5 (2d,3H). |

| Bsp. Nr. | $R^7$ | $R^8$ | $R^9$ | $[\alpha]_D^{24}(CH_2Cl_2)$ | $^1H$-NMR ($CDCl_3$); δ-Werte (ppm) |
|---|---|---|---|---|---|
| 28 | H | $-(CH_2)_3-N(CH_3)_2$ | $C_6H_5-CH_2-O-$ | -23° (c = 0,392) | 9,0 (m,1H); 7,4 (s,5H); 5,2-4,9 (m,2H); 5,1-4,6 (m,2H); 3,8-3,2 (m,6H); 2,6-1,6 (m,12H); 2,25 (s,6H). |
| 29 | H | $-(CH_2)_2-C_6H_5$ | $(C_6H_5)_2CH-CH_2-$ | -51,1° (c = 0,47) | 7,4-7,1 (m,10H); 5,0-4,5 (m,2H); 3,9-3,3 (2m,2H); 3,7-3,5 (m,4H); 3,15-2,9 (m,2H); 2,85 (t,2H); 2,3-1,7 (m,10H). |
| 30 | H | $-CH_2-$ 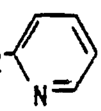 | $(C_6H_5)_2CH-CH_2-$ | -60,2° (c = 0,382) | 8,5 (d,1H); 8,1 (m,1H); 7,65 (dt,1H); 7,3-7,1 (m,11H); 5,1 (m,1H); 4,7-4,5 (m,5H); 3,9 (m,1H); 3,4-3,2 (m,2H); 3,3 (m,1H); 3,0 (m,2H); 2,3-1,7 (m,8H). |
| 31 | H | $-CH_2-C_6H_5$ | $(C_6H_5)_2CH-CH_2$ | -47,7° (c = 0,342) | 7,7 (m,1H); 7,4-7,1 (m,10H); 5,0 (m,1H); 4,7-4,3 (m,3H); 3,9-2,8 (m,4H); 2,3-1,7 (m,8H). |

| Bsp. Nr. | $R^7$ | $R^8$ | $R^9$ | $[\alpha]_D^{24}(CH_2Cl_2)$ | $^1$H-NMR ($CDCl_3$); δ-Werte (ppm) |
|---|---|---|---|---|---|
| 32 | H | $CH_3$ ⋮ $-CH-C_6H_5$ | $(C_6H_5)_2CH-CH_2-$ | -29,2° (c = 0,048) | 7,7 (d,1H); 7,4-7,1 (m,10H); 5,15 (m,1H); 5,0 (m,1H); 4,65 (m,1H); 4,55 (m,1H); 3,9-2,9 (m,4H); 2,3-1,7 (m,8H). |
| 33 | H | $-CH_2$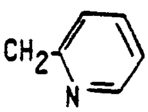 | $CH_3O$- -$(CH_2)_3$-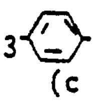 | -43,2° (c = 0,412) | 8,5 (d,1H); 8,1 (m,1H); 7,65 (dt,1H); 7,25 (m,2H); 7,1-6,7 (m,4H); 5,1 (m,1H); 4,65 (m,2H); 4,0-3,3 (m,3H); 3,8 (s,3H); 2,6 (t,2H); 2,4-1,8 (m,12H). |
| 34 | $-(CH_2)_4-$ | | $C_6H_5-CH_2-O-$ | -11,9° (c = 0,404) | 7,4-7,2 (m,5H); 5,2-4,9 (m,2H); 4,8-3,8 (m,2H); 3,7-3,3 (m,8H): 2,4-1,7 (m,12H). |
| 35 | $-(CH_2)_2-O-(CH_2)_2-$ | | $C_6H_5-CH_2-O-$ | -20,7° (c = 0,434) | 7,4-7,2 (m,5H); 5,2-4,9 (m,2H); 5,1-4,35 (m,2H); 3,9-3,3 (m,12H); 2,3-1,8 (m,8H). |

| Bsp. Nr. | $R^7$ | $R^8$ | $R^9$ | $[\alpha]_D^{24}(CH_2Cl_2)$ | $^1H$-NMR ($CDCl_3$); δ-Werte (ppm) |
|---|---|---|---|---|---|
| 36 | $-(CH_2)_2-N(CH_3)-(CH_2)_2-$ | | $C_6H_5-CH_2-O-$ | -27,2° (c = 0,492) | 7,4-7,2 (m,5H); 5,2-4,9 (m,2H); 5,1-4,6 (m,2H); 3,95-3,3 (m,8H); 2,6-1,8 (m,12H); 2,3 (2s,3H). |
| 37 | 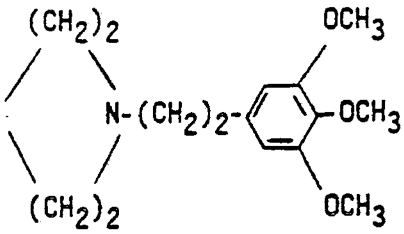 | | $C_6H_5-CH_2-O-$ | -21,7° (c = 0,552) | 7,4-7,2 (m,5H); 6,42-6,4 (2s,2H); 5,2-4,9 (m,2H); 5,1-4,6 (m,2H); 3,85, 3,83, 3,80 (3s,9H); 3,9-3,3 (m,8H); 2,8-1,8 (m,16H). |

**Ansprüche**

1. Verbindung der Formel I

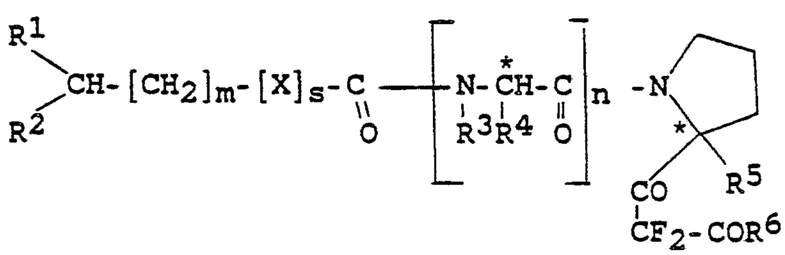 (I)

in welcher
$R^1$ Wasserstoff; ($C_1$-$C_6$)-Alkyl oder ($C_6$-$C_{12}$)-Aryl, das gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist, bedeutet;
$R^2$ Wasserstoff; ($C_1$-$C_6$)-Alkyl; ($C_6$-$C_{12}$)-Aryl; ($C_6$-$C_{12}$)-Aryloxy; ($C_7$-$C_{13}$)-Aroyl; Hydroxy oder ($C_1$-$C_4$)-Alkoxy bedeutet, wobei Aryl, Aryloxy und Aroyl jeweils gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist; oder
$R^1$ und $R^2$ zusammen für Benzyliden stehen, worin der Phenylring gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy,

Amino, $(C_1-C_4)$-Alkylamino, und Di-$(C_1-C_4)$-alkylamino oder ein ($C_1$ oder $C_2$)-Alkylendioxy substituiert ist;

$R^3$ Wasserstoff; $(C_1-C_6)$-Alkyl; $C_6-C_{12}$)-Aryl-$(C_1-C_6)$-alkyl; $(C_5-C_9)$-Cycloalkyl; Indanyl oder Tetrahydronaphthyl bedeutet,

$R^4$ Wasserstoff; $(C_1-C_6)$-Alkyl, das gegebenenfalls durch Amino, $(C_1-C_6)$-Acylamino oder Benzoylamino monosubstituiert sein kann; $(C_2-C_6)$-Alkenyl; $(C_5-C_9)$-Cycloalkyl; $(C_5-C_9)$-Cycloalkenyl; $(C_5-C_7)$-Cycloalkyl-$(C_1-C_4)$-alkyl; $(C_6-C_{12})$-Aryl oder teilhydriertes $(C_6-C_{12})$-Aryl, die jeweils durch einen, zwei oder drei gleiche oder verschiedene Reste aus der Reihe $(C_1-C_4)$-Alkyl, ($C_1$ oder $C_2$)-Alkoxy und Halogen substituiert sein können; $(C_6-C_{12})$-Aryl-$(C_1-C_4)$-alkyl oder $(C_7-C_{13})$-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1-9 Ringatome, Kohlenstoffatome und 1 bis 2 Ringatome Schwefel-oder Sauerstoffatome und/oder wovon 1 bis 4 Ringatome Stickstoffatome darstellen, oder, falls von vorstehenden Definitionen noch nicht umfaßt, die gegebenenfalls geschützte Seitenkette einer natürlich vorkommenden $\alpha$-Aminosäure bedeutet; oder

$R^3$ und $R^4$ zusammen für $-[CH_2]_p$-stehen, worin p = 3, 4 oder 5 ist und worin eine Methylengruppe durch S oder O ersetzt sein kann;

$R^5$ Wasserstoff; $(C_1-C_6)$-Alkyl; $(C_6-C_{12})$-Aryl-$(C_1-C_5)$-alkyl, das im Arylteil gegebenenfalls durch einen, zwei oder drei gleiche oder verschiedene Reste aus der Reihe $(C_1-C_4)$-Alkyl, $(C_1-C_4)$-Alkoxy, Halogen, Nitro, Hydroxy, Amino, $(C_1-C_4)$-Alkylamino und Di-$(C_1-C_4)$-alkylamino, oder ein ($C_1$ oder $C_2$)-Alkylendioxy substituiert ist, bedeutet;

$R^6$ $(C_1-C_8)$-Alkyl; $(C_6-C_{12})$-Aryl; $(C_6-C_{12})$-Aryl-$(C_1-C_6)$-alkyl; Hydroxy; $(C_1-C_8)$-Alkoxy; $(C_6-C_{12})$-Aryloxy oder $(C_6-C_{12})$-Aryl-$(C_1-C_6)$-alkoxy bedeutet, wobei Aryl, Aralkyl, Aryloxy und Arylalkoxy jeweils im Arylteil durch einen, zwei oder drei gleiche oder verschiedene Reste aus der Reihe $(C_1-C_4)$-Alkyl, ($C_1$ oder $C_2$)-Alkoxy, Halogen und Nitro oder ein ($C_1$ oder $C_2$) Alkylendioxy substituiert sein können; oder

$R^6$ für ein Rest der Formel $-NR^7R^8$ steht;

$R^7$ und $R^8$ gleich oder verschieden sind und Wasserstoff, $(C_1-C_8)$-Alkyl; $(C_4-C_{10})$-Cycloalkyl; $(C_1-C_4)$-Alkyl-$(C_4-C_{10})$-Cycloalkyl; $(C_6-C_{12})$-Aryl, $(C_6-C_{12})$-Aryl-$(C_1-C_6)$-alkyl; 2-, 3-oder 4-Pyridyl-$(C_1-C_4)$-alkyl; Amino-$(C_1-C_8)$-alkyl; $(C_1-C_4)$-Alkylamino-$(C_1-C_8)$-alkyl; Di-$(C_1-C_4)$-alkylamino-$(C_1-C_8)$-alkyl; Hydroxy-$(C_1-C_8)$-alkyl oder $(C_1-C_4)$-Alkoxy-$(C_1-C_8)$-alkyl bedeuten, wobei Aryl und Aralkyl jeweils im Arylteil durch einen, zwei oder drei gleiche oder verschiedene Reste aus der Reihe $(C_1-C_4)$-Alkyl, ($C_1$ oder $C_2$)-Alkoxy, $(C_1-C_4)$-Alkylamino, Di-$(C_1-C_4)$-alkylamino, Halogen, Hydroxy und Amino, oder ein ($C_1$ oder $C_2$)-Alkylendioxy substituiert sein können; oder

$R^7$ und $R^8$ zusammen mit dem diese tragenden Stickstoffatom einen 4-bis 12-gliedrigen, gesättigten oder teilweise ungesättigten mono-oder bicyclischen heterocyclischen Ring bilden, der als Ringatome 2-11 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe $(C_1-C_8)$-Alkyl, Phenyl, Phenyl-$(C_1-C_6)$-alkyl, Benzoyl, Phenyl-$(C_2-C_4)$-alkanoyl, 2-oder 3-Furoyl, 2-, 3-oder 4-Pyridyl und 2-, 4-oder 5-Pyrimidinyl substituiert ist, wobei Phenyl, Phenylalkyl, Benzoyl und Phenylalkanoyl ihrerseits jeweils im Phenylteil durch einen, zwei oder drei gleiche oder verschiedene Reste aus der Reihe $(C_1-C_4)$-Alkyl, ($C_1$ oder $C_2$)-Alkoxy, Halogen und Nitro oder ein ($C_1$ oder $C_2$)-Alkylendioxy substituiert sein können;

X Sauerstoff, Imino oder N-$(C_1-C_8)$-Alkylimino;

m = 0, 1, 2, 3, 4 oder 5 ist;

n = 0, 1 oder 2 ist und

s = 0 oder 1 ist,

sowie deren physiologisch verträgliche Salze, falls solche gebildet werden können.

2. Verbindung der Formel I gemäß Anspruch 1, in welcher

$R^1$ Wasserstoff; $(C_1-C_4)$-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Fluor, Chlor, Brom, Nitro, Hydroxy, Amino, Methylamino und Dimethylamino, oder ein Methylendioxy substituiert ist, oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, drei Methoxy, oder ein Methylendioxy substituiert ist;

$R^3$ Wasserstoff; $(C_1-C_4)$-Alkyl; Benzyl; Phenethyl; Cyclopentyl; Cyclohexyl oder Indanyl bedeutet;

$R^4$ Wasserstoff oder die gegebenenfalls geschützte Seitenkette einer natürlich vorkommenden $\alpha$-Amino-

säure bedeutet, oder

$R^3$ und $R^4$ zusammen für einen Rest - $[CH_2]_p$ -stehen, der wie im Anspruch 1 definiert ist und worin p = 3 oder 4 ist;

$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;

$R^6$ $(C_1-C_5)$-Alkoxy; Phenoxy; Phenyl-$(C_1-C_4)$-alkoxy oder Hydroxy bedeutet, wobei Phenoxy und Phenylalkoxy im Phenylrest jeweils durch einen oder zwei gleiche oder verschiedene Reste aus der Reihe Methyl, Ethyl, Methoxy, Fluor, Chlor und Brom oder ein Methylendioxy substituiert sein können; oder

$R^6$ für einen Rest der Formel $-NR^7R^8$ steht, worin $R^7$ und $R^8$ gleich oder verschieden sind und Wasserstoff; $(C_1-C_6)$-Alkyl; $(C_5-C_8)$-Cycloalkyl; ($C_1$ oder $C_2$)-Alkyl-$(C_5-C_8)$-cycloalkyl; Phenyl; Phenyl-$(C_1-C_4)$-alkyl; 2-, 3-oder 4-Pyridyl-$(C_1-C_4)$-alkyl; Amino-$(C_1-C_4)$-alkyl; ($C_1$ oder $C_2$)-Alkylamino-$(C_1-C_4)$-alkyl; Di-($C_1$ oder $C_2$)-alkylamino-$(C_1-C_4)$-alkyl; Hydroxy-$(C_1-C_4)$-alkyl oder ($C_1$ oder $C_2$)-Alkoxy-$(C_1-C_4)$-alkyl bedeuten, worin Phenyl und Phenylalkyl jeweils im Phenylteil durch einen oder zwei gleiche oder verschiedene Reste aus der Reihe Methyl, Ethyl, Methoxy, Fluor, Chlor, Brom, Amino, Methylamino und Dimethylamino, oder ein Methylendioxy substituiert sein können; oder

$R^7$ und $R^8$ zusammen mit dem diese tragenden Stickstoffatom einen 5-bis 9-gliedrigen heterocyclischen Ring bilden, der als Ringglieder 3-8 Kohlenstoffatome und gegebenenfalls ein weiteres Stickstoffatom oder ein Sauerstoffatom enthält und der gegebenenfalls durch $(C_1-C_4)$-Alkyl, Phenyl, Phenyl-$(C_1-C_4)$-alkyl, Benzoyl, Phenyl-($C_2$ oder $C_3$)-alkanoyl, 2-oder 3-Furoyl, 2-, 3-oder 4-Pyridyl oder 2-, 4-oder 5-Pyrimidinyl monosubstituiert ist, wobei Phenyl, Phenylalkyl, Benzoyl und Phenylalkanoyl ihrerseits jeweils im Phenylteil durch einen oder zwei gleiche oder verschiedene Reste aus der Reihe Methyl, Ethyl, Methoxy, Fluor, Chlor und Brom oder ein Methylendioxy substituiert sein können;

X Sauerstoff bedeutet;

m = 0, 1, 2, 3, 4 oder 5 ist;

n = 0 oder 1 ist und

s = 0 oder 1 ist,

sowie deren physiologisch verträgliche Salze.

3. Verbindung der Formel I gemäß Anspruch 1 oder 2, in welcher

$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 2,3-, 2,4-, 2,5-, 2,6-, 3,4-oder 3,5-Dimethoxyphenyl bedeutet;

$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder

$R^1$ und $R^2$ zusammen für Benzyliden; o-, m-oder p-Methylbenzyliden; o-, m-oder p-Methoxybenzyliden oder 2,3-, 2,4-, 2,5-, 2,6, 3,4-oder 3,5-Dimethoxybenzyliden stehen;

$R^3$ Wasserstoff oder Methyl bedeutet;

$R^4$ Wasserstoff, Methyl, n-Propyl, Isopropyl, n-Butyl, Isobutyl, sec.-Butyl oder Benzyl bedeutet; oder

$R^3$ und $R^4$ zusammen für - $[CH_2]_3$ -stehen;

$R^5$ Wasserstoff oder Methyl bedeutet;

$R^6$ Methoxy; Ethoxy; Propoxy; Isopropoxy; tert.-Butoxy; n-Butoxy; Benzyloxy; 4-Methoxybenzyloxy; 4-Chlorbenzyloxy; 3,4-Dimethoxybenzyloxy; Phenethyloxy oder einen Rest der Formel $-NR^7R^8$ bedeutet;

$R^7$ und $R^8$ gleich oder verschieden sind und Wasserstoff, $(C_1-C_6)$-Alkyl; $(C_5-C_8)$-Cycloalkyl; Phenyl; Phenyl-$(C_1-C_4)$-alkyl, 2-, 3-oder 4-Pyridylmethyl; 2-, 3-oder 4-Pyridylethyl; Amino$(C_1-C_4)$-alkyl; Methylamino-$(C_1-C_4)$-alkyl; Dimethylamino-$(C_1-C_4)$-alkyl; Hydroxy-$(C_1-C_4)$-alkyl oder Methoxy-$(C_1-C_4)$-alkyl bedeuten, wobei Phenyl und Phenylalkyl jeweils im Phenylrest durch einen oder zwei gleiche oder verschiedene Reste aus der Reihe Methyl, Methoxy, Fluor, Chlor, Brom, Amino, Methylamino und Dimethylamino, oder ein Methylendioxy substituiert sein können; oder

$R^7$ und $R^8$ zusammen mit dem diese tragenden Stickstoffatom für Pyrrolidino, Piperidino, Tetrahydropyridino, Morpholino, Azepino, N,N-Heptamethylenimino, Piperazino oder Homopiperazino stehen, wobei die genannten Heterocyclenreste durch $(C_1-C_4)$-Alkyl, Phenyl, Phenyl-$(C_1-C_4)$-alkyl, Benzoyl, Phenyl-($C_2$ oder $C_3$)-alkanoyl oder 2-, 3-oder 4-Pyridyl monosubstituiert sein können und worin Phenyl, Phenylalkyl, Benzoyl und Phenylalkanoyl ihrerseits im Phenylteil durch einen oder zwei gleiche oder verschiedene Reste aus der Reihe Methyl, Methoxy, Fluor, Chlor oder Brom substituiert sein können;

X Sauerstoff bedeutet;
m = 0, 1, 2, 3, 4 oder 5 ist;
n = 0 oder 1 ist und
s = 0 oder 1 ist,
sowie deren physiologisch verträgliche Salze.

4. Verbindung der Formel I gemäß einem der Ansprüche 1 bis 3, in welcher
$R^2$ Wasserstoff bedeutet;
$R^3$ und $R^4$ zusammen für - $[CH_2]_3$ -stehen;
$R^5$ Wasserstoff bedeutet;
X Sauerstoff bedeutet;
m = 1, 2, 3 oder 4 ist;
n = 1 ist und
s = 0 oder 1 ist,
sowie deren physiologisch verträgliche Salze.

5. Verbindung der Formel I gemäß einem der Ansprüche 1 bis 3, in welcher
$R^5$ Wasserstoff bedeutet;
X Sauerstoff bedeutet;
m = 1, 2, 3 oder 4 ist;
n = 0 ist und
s = 0 oder 1 ist,
sowie deren physiologisch verträgliche Salze.

6. Verfahren zur Herstellung einer Verbindung der Formel I gemäß einem der Ansprüche 1 bis 5, dadurch gekennzeichnet, daß man eine Verbindung der Formel IV

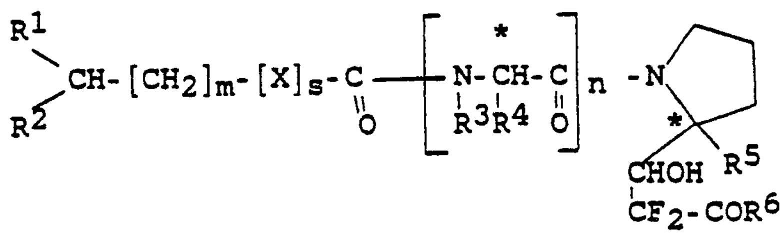

(IV)

in welcher $R^1$, $R^2$, $R^3$, $R^4$, $R^5$ und $R^6$, X, m, n und s die gleiche Bedeutung wie in Formel I haben, oxidiert und die so erhaltene Verbindung gegebenenfalls in ihre physiologisch verträglichen Salze überführt.

7. Verbindung gemäß einem der Ansprüche 1 bis 5 zur Anwendung als Heilmittel.

8. Verbindung gemäß einem der Ansprüche 1 bis 5 zur Anwendung als Prolyl-Endopeptidase-Hemmer.

9. Pharmazeutische Zubereitung, enthaltend eine Verbindung gemäß einem der Ansprüche 1 bis 5.

10. Verfahren zur Herstellung einer Zubereitung gemäß Anspruch 9, dadurch gekennzeichnet, daß man den Wirkstoff zusammen mit einem physiologisch annehmbaren Träger und gegebenenfalls weiteren Zusatz-, Hilfs und/oder Konservierungsstoffen in eine geeignete Darreichungsform bringt.

Patentansprüche für die folgenden Vertragstaaten: ES und GR:

1. Verfahren zur Herstellung einer Verbindung der Formel I

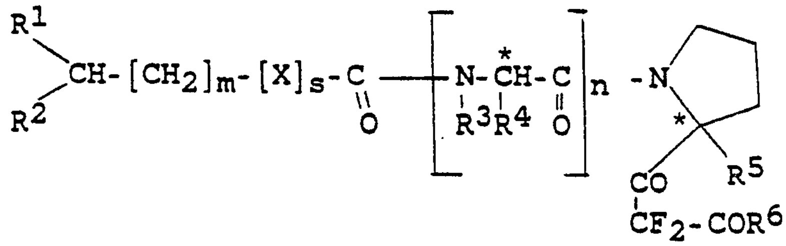

(I)

in welcher

$R^1$ Wasserstoff; ($C_1$-$C_6$)-Alkyl oder ($C_6$-$C_{12}$)-Aryl, das gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist, bedeutet;

$R^2$ Wasserstoff; ($C_1$-$C_6$)-Alkyl; ($C_6$-$C_{12}$)-Aryl; ($C_6$-$C_{12}$)-Aryloxy; ($C_7$-$C_{13}$)-Aroyl; Hydroxy oder ($C_1$-$C_4$)-Alkoxy bedeutet, wobei Aryl, Aryloxy und Aroyl jeweils gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist; oder

$R^1$ und $R^2$ zusammen für Benzyliden stehen, worin der Phenylring gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino, und Di-($C_1$-$C_4$)-alkylamino oder ein ($C_1$ oder $C_2$)-Alkylendioxy substituiert ist;

$R^3$ Wasserstoff; ($C_1$-$C_6$)-Alkyl; ($C_6$-$C_{12}$)-Aryl-($C_1$-$C_6$)-alkyl; ($C_5$-$C_9$)-Cycloalkyl; Indanyl oder Tetrahydronaphthyl bedeutet,

$R^4$ Wasserstoff; ($C_1$-$C_6$)-Alkyl, das gegebenenfalls durch Amino, ($C_1$-$C_6$)-Acylamino oder Benzoylamino monosubstituiert sein kann; ($C_2$-$C_6$)-Alkenyl; ($C_5$-$C_9$)-Cycloalkyl; ($C_5$-$C_9$)-Cycloalkenyl; ($C_5$-$C_7$)-Cycloalkyl-($C_1$-$C_4$)-alkyl; ($C_6$-$C_{12}$)-Aryl oder teilhydriertes ($C_6$-$C_{12}$)-Aryl, die jeweils durch einen, zwei oder drei gleiche oder verschiedene Reste aus der Reihe ($C_1$-$C_4$)-Alkyl, ($C_1$ oder $C_2$)-Alkoxy und Halogen substituiert sein können; ($C_6$-$C_{12}$)-Aryl-($C_1$-$C_4$)-alkyl oder ($C_7$-$C_{13}$)-Aroyl-($C_1$ oder $C_2$)-alkyl, die beide wie vorstehend definiert im Arylrest substituiert sein können; einen mono-bzw. bicyclischen Heterocyclen-Rest mit 5 bis 7 bzw. 8 bis 10 Ringatomen, wovon 1-9 Ringatome, Kohlenstoffatome und 1 bis 2 Ringatome Schwefel-oder Sauerstoffatome und/oder wovon 1 bis 4 Ringatome Stickstoffatome darstellen, oder, falls von vorstehenden Definitionen noch nicht umfaßt, die gegebenenfalls geschützte Seitenkette einer natürlich vorkommenden $\alpha$-Aminosäure bedeutet; oder

$R^3$ und $R^4$ zusammen für -$[CH_2]_p$-stehen, worin p = 3, 4 oder 5 ist und worin eine Methylengruppe durch S oder O ersetzt sein kann;

$R^5$ Wasserstoff; ($C_1$-$C_6$)-Alkyl; ($C_6$-$C_{12}$)-Aryl-($C_1$-$C_5$)-alkyl, das im Arylteil gegebenenfalls durch einen, zwei oder drei gleiche oder verschiedene Reste aus der Reihe ($C_1$-$C_4$)-Alkyl, ($C_1$-$C_4$)-Alkoxy, Halogen, Nitro, Hydroxy, Amino, ($C_1$-$C_4$)-Alkylamino und Di-($C_1$-$C_4$)-alkylamino, oder ein ($C_1$ oder $C_2$)-Alkylendioxy substituiert ist, bedeutet;

$R^6$ ($C_1$-$C_8$)-Alkyl; ($C_6$-$C_{12}$)-Aryl; ($C_6$-$C_{12}$)-Aryl-($C_1$-$C_6$)-alkyl; Hydroxy; ($C_1$-$C_8$)-Alkoxy; ($C_6$-$C_{12}$)-Aryloxy oder ($C_6$-$C_{12}$)-Aryl-($C_1$-$C_6$)-alkoxy bedeutet, wobei Aryl, Aralkyl, Aryloxy und Arylalkoxy jeweils im Arylteil durch einen, zwei oder drei gleiche oder verschiedene Reste aus der Reihe ($C_1$-$C_4$)-Alkyl, ($C_1$ oder $C_2$)-Alkoxy, Halogen und Nitro oder ein ($C_1$ oder $C_2$) Alkylendioxy substituiert sein können; oder

$R^6$ für ein Rest der Formel $-NR^7R^8$ steht;

$R^7$ und $R^8$ gleich oder verschieden sind und Wasserstoff, ($C_1$-$C_8$)-Alkyl; ($C_4$-$C_{10}$)-Cycloalkyl; ($C_1$-$C_4$)-Alkyl-($C_4$-$C_{10}$)-Cycloalkyl; ($C_6$-$C_{12}$)-Aryl, ($C_6$-$C_{12}$)-Aryl-($C_1$-$C_6$)-alkyl; 2-, 3-oder 4-Pyridyl-($C_1$-$C_4$)-alkyl; Amino-($C_1$-$C_8$)-alkyl; ($C_1$-$C_4$)-Alkylamino-($C_1$-$C_8$)-alkyl; Di-($C_1$-$C_4$)-alkylamino-($C_1$-$C_8$)-alkyl; Hydroxy-($C_1$-$C_8$)-alkyl oder ($C_1$-$C_4$)-Alkoxy-($C_1$-$C_8$)-alkyl bedeuten, wobei Aryl und Aralkyl jeweils im Arylteil durch einen, zwei oder drei gleiche oder verschiedene Reste aus der Reihe ($C_1$-$C_4$)-Alkyl, ($C_1$ oder $C_2$)-Alkoxy, ($C_1$-$C_4$)-Alkylamino, Di-($C_1$-$C_4$)-alkylamino, Halogen, Hydroxy und Amino, oder ein ($C_1$ oder $C_2$)-Alkylendioxy substituiert sein können; oder

$R^7$ und $R^8$ zusammen mit dem diese tragenden Stickstoffatom einen 4-bis 12-gliedrigen, gesättigten oder teilweise ungesättigten mono-oder bicyclischen heterocyclischen Ring bilden, der als Ringatome 2-11 Kohlenstoffatome und gegebenenfalls 1 oder 2 weitere gleiche oder verschiedene Heteroatome aus der Reihe Sauerstoff, Schwefel und Stickstoff enthält und der gegebenenfalls durch einen Rest aus der Reihe ($C_1$-$C_8$)-Alkyl, Phenyl, Phenyl-($C_1$-$C_6$)-alkyl, Benzoyl, Phenyl-($C_2$-$C_4$)-alkanoyl, 2-oder 3-Furoyl, 2-, 3-oder 4-Pyridyl und 2-, 4-oder 5-Pyrimidinyl substituiert ist, wobei Phenyl, Phenylalkyl, Benzoyl und Phenylalkanoyl ihrerseits jeweils im Phenylteil durch einen, zwei oder drei gleiche oder verschiedene Reste aus der Reihe ($C_1$-$C_4$)-Alkyl, ($C_1$ oder $C_2$)-Alkoxy, Halogen und Nitro oder ein ($C_1$ oder $C_2$)-Alkylendioxy substituiert sein können;

X Sauerstoff, Imino oder N-($C_1$-$C_8$)-Alkylimino;

m = 0, 1, 2, 3, 4 oder 5 ist;

n = 0, 1 oder 2 ist und

s = 0 oder 1 ist,

sowie deren physiologisch verträgliche Salze, falls solche gebildet werden können, dadurch gekennzeichnet, daß man eine Verbindung der Formel IV

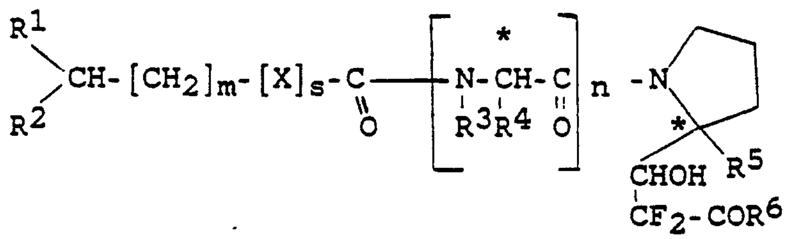

(IV)

in welcher $R^1$, $R^2$, $R^3$, $R^4$, $R^5$ und $R^6$, X, m, n und s die gleiche Bedeutung wie in Formel I haben, oxidiert und die so erhaltene Verbindung gegebenenfalls in ihre physiologisch verträglichen Salze überführt.

2. Verfahren gemäß Anspruch 1, dadurch gekennzeichnet, daß man eine Verbindung der Formel I herstellt, in welcher

$R^1$ Wasserstoff; $(C_1-C_4)$-Alkyl oder Phenyl, das gegebenenfalls durch einen oder zwei gleiche oder verschiedene Reste aus der Reihe Methyl, Ethyl, Methoxy, Fluor, Chlor, Brom, Nitro, Hydroxy, Amino, Methylamino und Dimethylamino, oder drei Methoxy, oder ein Methylendioxy substituiert ist, bedeutet;

$R^2$ Wasserstoff; Phenyl; Phenoxy; Benzoyl; Hydroxy oder Methoxy bedeutet, wobei Phenyl, Phenoxy und Benzoyl jeweils gegebenenfalls durch einen oder zwei gleiche oder verschiedene Reste aus der Reihe Methyl, Ethyl, Fluor, Chlor, Brom, Nitro, Hydroxy, Amino, Methylamino und Dimethylamino, oder ein Methylendioxy substituiert ist, oder

$R^1$ und $R^2$ zusammen für Benzyliden stehen, worin der Phenylrest gegebenenfalls durch einen oder zwei gleiche oder verschiedene Reste aus der Reihe Methyl, Ethyl, Methoxy, Fluor, Chlor, Brom und Hydroxy, drei Methoxy, oder ein Methylendioxy substituiert ist;

$R^3$ Wasserstoff; $(C_1-C_4)$-Alkyl; Benzyl; Phenethyl; Cyclopentyl; Cyclohexyl oder Indanyl bedeutet;

$R^4$ Wasserstoff oder die gegebenenfalls geschützte Seitenkette einer natürlich vorkommenden α-Aminosäure bedeutet, oder

$R^3$ und $R^4$ zusammen für einen Rest - $[CH_2]_p$ -stehen, der wie im Anspruch 1 definiert ist und worin p = 3 oder 4 ist;

$R^5$ Wasserstoff; Methyl; Ethyl; Benzyl oder Phenethyl, welche jeweils im Phenylrest gegebenenfalls durch einen, zwei oder drei gleiche oder verschiedene Reste aus der Reihe Methoxy, Fluor, Chlor und Brom, oder ein Methylendioxy substituiert sind, bedeutet;

$R^6$ $(C_1-C_5)$-Alkoxy; Phenoxy; Phenyl-$(C_1-C_4)$-alkoxy oder Hydroxy bedeutet, wobei Phenoxy und Phenylalkoxy im Phenylrest jeweils durch einen oder zwei gleiche oder verschiedene Reste aus der Reihe Methyl, Ethyl, Methoxy, Fluor, Chlor und Brom oder ein Methylendioxy substituiert sein können; oder

$R^6$ für einen Rest der Formel $-NR^7R^8$ steht, worin $R^7$ und $R^8$ gleich oder verschieden sind und Wasserstoff; $(C_1-C_6)$-Alkyl; $(C_5-C_8)$-Cycloalkyl; ($C_1$ oder $C_2$)-Alkyl-$(C_5-C_8)$-cycloalkyl; Phenyl; Phenyl-$(C_1-C_4)$-alkyl; 2-, 3- oder 4-Pyridyl-$(C_1-C_4)$-alkyl; Amino-$(C_1-C_4)$-alkyl; ($C_1$ oder $C_2$)-Alkylamino-$(C_1-C_4)$-alkyl; Di-($C_1$ oder $C_2$)-alkylamino-$(C_1-C_4)$-alkyl; Hydroxy-$(C_1-C_4)$-alkyl oder ($C_1$ oder $C_2$)-Alkoxy-$(C_1-C_4)$-alkyl bedeuten, worin Phenyl und Phenylalkyl jeweils im Phenylteil durch einen oder zwei gleiche oder verschiedene Reste aus der Reihe Methyl, Ethyl, Methoxy, Fluor, Chlor, Brom, Amino, Methylamino und Dimethylamino, oder ein Methylendioxy substituiert sein können; oder

$R^7$ und $R^8$ zusammen mit dem diese tragenden Stickstoffatom einen 5-bis 9-gliedrigen heterocyclischen Ring bilden, der als Ringglieder 3-8 Kohlenstoffatome und gegebenenfalls ein weiteres Stickstoffatom oder ein Sauerstoffatom enthält und der gegebenenfalls durch $(C_1-C_4)$-Alkyl, Phenyl, Phenyl-$(C_1-C_4)$-alkyl, Benzoyl, Phenyl-($C_2$ oder $C_3$)-alkanoyl, 2-oder 3-Furoyl, 2-, 3-oder 4-Pyridyl oder 2-, 4-oder 5-Pyrimidinyl monosubstituiert ist, wobei Phenyl, Phenylalkyl, Benzoyl und Phenylalkanoyl ihrerseits jeweils im Phenylteil durch einen oder zwei gleiche oder verschiedene Reste aus der Reihe Methyl, Ethyl, Methoxy, Fluor, Chlor und Brom oder ein Methylendioxy substituiert sein können;

X Sauerstoff bedeutet;

m = 0, 1, 2, 3, 4 oder 5 ist;

n = 0 oder 1 ist und

s = 0 oder 1 ist,

sowie deren physiologisch verträgliche Salze.

3. Verfahren gemäß Anspruch 1 oder 2, dadurch gekennzeichnet, daß man eine Verbindung der Formel I herstellt, in welcher

$R^1$ Wasserstoff; Methyl; Ethyl; Propyl; Isopropyl; Phenyl; o-, m-oder p-Tolyl; o-, m-oder p-Chlorphenyl; o-, m-oder p-Fluorphenyl; o-, m-oder p-Methoxyphenyl oder 2,3-, 2,4-, 2,5-, 2,6-, 3,4-oder 3,5-Dimethoxyphenyl bedeutet;

$R^2$ Wasserstoff; Phenyl; o-, m-oder p-Tolyl; o-, m-oder p-Chlorphenyl; o-, m-oder p-Fluorphenyl; o-, m-oder p-Methoxyphenyl; 2,3-, 2,4-, 2,5-, 2,6-, 3,4-oder 3,5-Dimethoxyphenyl; Phenoxy; o-, m-oder p-Tolyloxy; o-, m-oder p-Chlorphenoxy; o-, m-oder p-Fluorphenoxy; o-, m-oder p-Methoxyphenoxy; 2,3-, 2,4-, 2,5-, 2,6-, 3,4-oder 3,5-Dimethoxyphenoxy; Benzoyl; o-, m-oder p-Toluoyl; o-, m-oder p-Chlorbenzoyl; o-, m-oder p-Fluorbenzoyl; o-, m-oder p-Methoxybenzoyl; 2,3-, 2,4-, 2,5-, 2,6-, 3,4-oder 3,5-Dimethoxybenzoyl; Hydroxy oder Methoxy bedeutet; oder

$R^1$ und $R^2$ zusammen für Benzyliden; o-, m-oder p-Methylbenzyliden; o-, m-oder p-Methoxybenzyliden oder 2,3-, 2,4-, 2,5-, 2,6, 3,4-oder 3,5-Dimethoxybenzyliden stehen;

$R^3$ Wasserstoff oder Methyl bedeutet;

$R^4$ Wasserstoff, Methyl, n-Propyl, Isopropyl, n-Butyl, Isobutyl, sec.-Butyl oder Benzyl bedeutet; oder

$R^3$ und $R^4$ zusammen für - $[CH_2]_3$ -stehen;

$R^5$ Wasserstoff oder Methyl bedeutet;

$R^6$ Methoxy; Ethoxy; Propoxy; Isopropoxy; tert.-Butoxy; n-Butoxy; Benzyloxy; 4-Methoxybenzyloxy; 4-Chlorbenzyloxy; 3,4-Dimethoxybenzyloxy; Phenethyloxy oder einen Rest der Formel $-NR^7R^8$ bedeutet;

$R^7$ und $R^8$ gleich oder verschieden sind und Wasserstoff, $(C_1-C_6)$-Alkyl; $(C_5-C_8)$-Cycloalkyl; Phenyl; Phenyl-$(C_1-C_4)$-alkyl, 2-, 3-oder 4-Pyridylmethyl; 2-, 3-oder 4-Pyridylethyl; Amino$(C_1-C_4)$-alkyl; Methylamino-$(C_1-C_4)$-alkyl; Dimethylamino-$(C_1-C_4)$-alkyl; Hydroxy-$(C_1-C_4)$-alkyl oder Methoxy-$(C_1-C_4)$-alkyl bedeuten, wobei Phenyl und Phenylalkyl jeweils im Phenylrest durch einen oder zwei gleiche oder verschiedene Reste aus der Reihe Methyl, Methoxy, Fluor, Chlor, Brom, Amino, Methylamino und Dimethylamino, oder ein Methylendioxy substituiert sein können; oder

$R^7$ und $R^8$ zusammen mit dem diese tragenden Stickstoffatom für Pyrrolidino, Piperidino, Tetrahydropyridino, Morpholino, Azepino, N,N-Heptamethylenimino, Piperazino oder Homopiperazino stehen, wobei die genannten Heterocyclenreste durch $(C_1-C_4)$-Alkyl, Phenyl, Phenyl-$(C_1-C_4)$-alkyl, Benzoyl, Phenyl-$(C_2$ oder $C_3)$-alkanoyl oder 2-, 3-oder 4-Pyridyl monosubstituiert sein können und worin Phenyl, Phenylalkyl, Benzoyl und Phenylalkanoyl ihrerseits im Phenylteil durch einen oder zwei gleiche oder verschiedene Reste aus der Reihe Methyl, Methoxy, Fluor, Chlor oder Brom substituiert sein können;

X Sauerstoff bedeutet;

m = 0, 1, 2, 3, 4 oder 5 ist;

n = 0 oder 1 ist und

s = 0 oder 1 ist,

sowie deren physiologisch verträgliche Salze.

4. Verfahren gemäß einem der Ansprüche 1 bis 3, dadurch gekennzeichnet, daß man eine Verbindung der Formel I herstellt,

in welcher

$R^2$ Wasserstoff bedeutet;

$R^3$ und $R^4$ zusammen für - $[CH_2]_3$ -stehen;

$R^5$ Wasserstoff bedeutet;

X Sauerstoff bedeutet;

m = 1, 2, 3 oder 4 ist;

n = 1 ist und

s = 0 oder 1 ist,

sowie deren physiologisch verträglichen Salze.

5. Verfahren gemäß einem der Ansprüche 1 bis 3, dadurch gekennzeichnet, daß man eine Verbindung der Formel I herstellt,

in welcher

$R^5$ Wasserstoff bedeutet;

X Sauerstoff bedeutet;

m = 1, 2, 3 oder 4 ist;

n = 0 ist und

s = 0 oder 1 ist,

sowie deren physiologisch veträglichen Salze.

6. Verfahren zur Herstellung einer Zubereitung enthaltend eine Verbindung gemäß einem der Anspruche 1 bis 5, dadurch gekennzeichnet, daß man den Wirkstoff zusammen mit einem physiologisch annehmbaren Träger und gegebenenfalls weiteren Zusatz-, Hilfs und/oder Konservierungsstoffen in eine geeignete Darreichungsform bringt.